**Europäisches Patentamt**

**European Patent Office**

**Office européen des brevets**

(19)

(11) Veröffentlichungsnummer: **0 101 870**

**A2**

(12) ## EUROPÄISCHE PATENTANMELDUNG

(21) Anmeldenummer: **83106967.9**

(22) Anmeldetag: **15.07.83**

(51) Int. Cl.³: **A 61 B 5/04**

(30) Priorität: **05.08.82 CH 4717/82**

(43) Veröffentlichungstag der Anmeldung:
**07.03.84 Patentblatt 84/10**

(84) Benannte Vertragsstaaten:
**CH DE FR GB IT LI NL**

(71) Anmelder: **KONTRON-HOLDING AG**
**Bernerstrasse Süd 169**
**CH-8048 Zürich(CH)**

(72) Erfinder: **Marangoni, Daniele, Dr.**
**Via Medici del Vascello n. 26**
**Mailand(IT)**

(74) Vertreter: **Körber, Wolfhart, Dr. et al,.**
**Patentanwälte Dipl.Ing.H.Mitscherlich,**
**Dipl.Ing.K.Gunschmann, Dr.rer.nat.W.Körber,**
**Dipl.Ing.J.Schmidt-Evers Steinsdorfstrasse 10**
**D-8000 München 22(DE)**

(54) **Tragbares Elektrokardiologiegerät.**

(57) Tragbares Elektrokardiologiegerät (1) mit Körperelektroden (7, 8, 11) zur Erfassung von Herzaktionssignalen, Verstärker (12) und Signalumformer zur Umformung der erfassten Signale in zur Aufzeichnung auf Magnetband geeignete Signale und einem Magnetbandaufzeichnungsteil, das sich dadurch auszeichnet, dass die Körperelektroden (7, 8) direkt am Gehäuse des Aufzeichnungsteils und zwar die Mess- und die Referenz-Elektrode an einer ersten Wand (6), die Erdungselektrode an einer von der ersten verschiedenen Wand (9) des Geräts (1) angeordnet sind und dass zusätzlich ein Mikrofon (5) zur Aufzeichnung einer gesprochenen Beschreibung der Symptome und dergleichen vorhanden ist. Das Gerät kann vom Patienten immer mitgeführt und jeweils während des Auftretens entsprechender Symptome zur Aufzeichnung einer diagnostisch relevanten EKG-Phase in Betrieb gesetzt werden.

Fig. 1

Fig. 2

EP 0 101 870 A2

**0101870**

Kontron Holding AG, Bernerstrasse Süd 169, 8048 Zürich


Ref. 6800/23




Tragbares Elektrokardiologiegerät




Die Erfindung betrifft ein tragbares Elektrokardiologiegerät mit Körperelektroden zur Erfassung von Herzaktionssignalen, Verstärker und Signalumformer zur Umformung der erfassten Signale in zur Aufzeichnung auf Magnetband geeignete Signale und einem Magnetbandaufzeichnungsteil.

Tragbare Elektrokardiologiegeräte dienen zur Erfassung und Aufzeichnung oder Speicherung von Herzaktionssignalen und kommen vor allem bei der Patientenüberwachung und für Belastungstests in der Arbeits- und Sportmedizin zum Einsatz. Je nach Einsatzgebiet und Zielrichtung der Ueberwachung werden Systeme verwendet, die sich teilweise grundlegend voneinander unterscheiden.

Bekannt sind Bandaufzeichnungsgeräte, die vom Patienten bzw. der Testperson getragen werden und die mit herkömmlichen Körperelektroden verbunden sind. Je nach dem Ueberwachungszweck können diese Geräte die Herzaktionssignale (EKG) während einer bestimmten Zeit, in der Regel 24 Stunden, auf Magnetband aufzeichnen. Diese Geräte werden nach dem Pionier auf diesem Gebiet, Dr. N. Holter,

Bu/25.5.83

benannt. Um den Aufwand bei der Auswertung der Aufzeichnung zu verringern, wurden diese Systeme dahingehend weiter entwickelt, dass das Aufzeichnungsgerät nur bei bestimmten Vorfällen, z.B. Herzarhythmien, eingeschaltet wird oder sich selbst einschaltet. Auf diese Weise kann auch die Einsatzdauer auf bis zu 72 Stunden ausgedehnt werden. Eine längere Einsatzdauer ist in der Regel nicht möglich, weil durch den Elektrodenkontakt eine zeitliche Begrenzung gegeben ist.

Die Auswertung des auf Magnetband aufgezeichneten EKG's erfolgt in der Regel durch den Arzt in einem speziellen Auswertgerät, in dem die Magnetbandaufzeichnung in eine optische Darstellung auf dem Bildschirm und/oder auf Papier übertragen wird.

Ein anderes System, das in den Vereinigten Staaten weit verbreitet zur Anwendung kommt, ist die telephonische EKG-Ueberwachung. Dabei setzt sich der Patient beim Auftreten eines Symptoms, beispielsweise einer Herzarhythmie, direkt über Telephon mit einem bei seinem Arzt installierten Aufzeichnungsgerät in Verbindung, um die von einem mitgeführten EKG-Verstärker erzeugten Signale über Telephon zu übertragen. Eine Weiterentwicklung dieses Systems besteht darin, dass der EKG-Verstärker noch mit einem Kurzzeitspeicher versehen ist, der zur vorübergehenden Speicherung der Signale dient, um die Abhängigkeit von der unmittelbaren Verfügbarkeit eines Telephons auszuschalten. Der Einsatz solcher Geräte ist nur dort möglich, wo ein entsprechendes telephonisches Uebertragungs- und Aufzeichnungssystem etabliert ist. Dies ist derzeit auf die Vereinigten Staaten beschränkt.

Durch die Uebertragung per Telephon ist die Genauigkeit des aufgezeichneten EKG sehr limitiert. Deshalb kann ein vereinfachter EKG-Verstärker mit geringer Bandbreite und vor allem eine vereinfachte Elektrodenanordnung zur Anwendung kommen. Es wurden daher Geräte vorgeschlagen, bei denen die Elektroden direkt am Gehäuse des EKG-Verstärkers angebracht sind. Mögliche Störungen durch die eng beieinanderliegenden Elektroden sind bei der geringen Qualität des Uebertragungssystems ohne Belang.

Der Erfindung liegt die Aufgabe zugrunde, ein tragbares Elektrokardiologiegerät bereitzustellen, das vom Patienten oder der Testperson über lange Zeit mitgeführt und jeweils bei Bedarf ohne spezielle Vorbereitung während bestimmter Symptomphasen zur Aufzeichung eingesetzt werden kann.

Erfindungsgemäss wird diese Aufgabe dadurch gelöst, dass bei einem Gerät der eingangs erwähnten Art die Elektroden direkt am Gehäuse des Aufzeichnungsteils angebracht sind.

Nach einer speziellen Ausführungsform des Geräts umfassen die Körperelektroden Mess-, Referenz- und Erdungselektroden, von denen die beiden ersteren an einer ersten Wand des Geräts im Abstand voneinander angebracht sind. Bei diesem Gerät ist die Erdungselektrode vorzugsweise an einer von der ersten verschiedenen Wand des Geräts angeordnet.

Nach einer weiteren Ausführungsform des Geräts ist eine der Elektroden mit einem Druckschalter integriert.

Nach wieder einer anderen Ausführungsform ist zusätzlich ein Mikrofon zur Aufzeichnung akustischer Signale, beispielsweise einer kurzen mündlichen Beschreibung der Symptome durch den Patienten, vorgesehen.

Eine bevorzugte Ausführungsform des erfindungsgemässen Geräts ist nachfolgend mit Bezug auf die beiliegenden Zeichnungen beschrieben.

Es zeigen

Fig. 1 zwei perspektivische Ansichten des Geräts.

Fig. 2 eine schematische Darstellung, wie das Gerät vom Patienten zur Erfassung der Herzaktionsignale gehalten wird,

Fig. 3 ein Blockschaltbild des EKG-Teils des Geräts,

Fig. 4 eine spezielle Ausführungsform einer Gehäuse-Elektrode mit einem integrierten Schalter im Schnitt.

Fig. 5 eine vergrösserte Darstellung der Elektrodenoberfläche,

Fig. 6 eine Ausführungsform der zweiten Gehäuseelektrode im Schnitt,

Fig. 7 eine schematische Darstellung der beim Arzt installierten Einrichtung zur Umformung der aufgezeichneten Signale,

Fig. 8 eine Alternative zu der in Fig. 6 gezeigten Einrichtung.

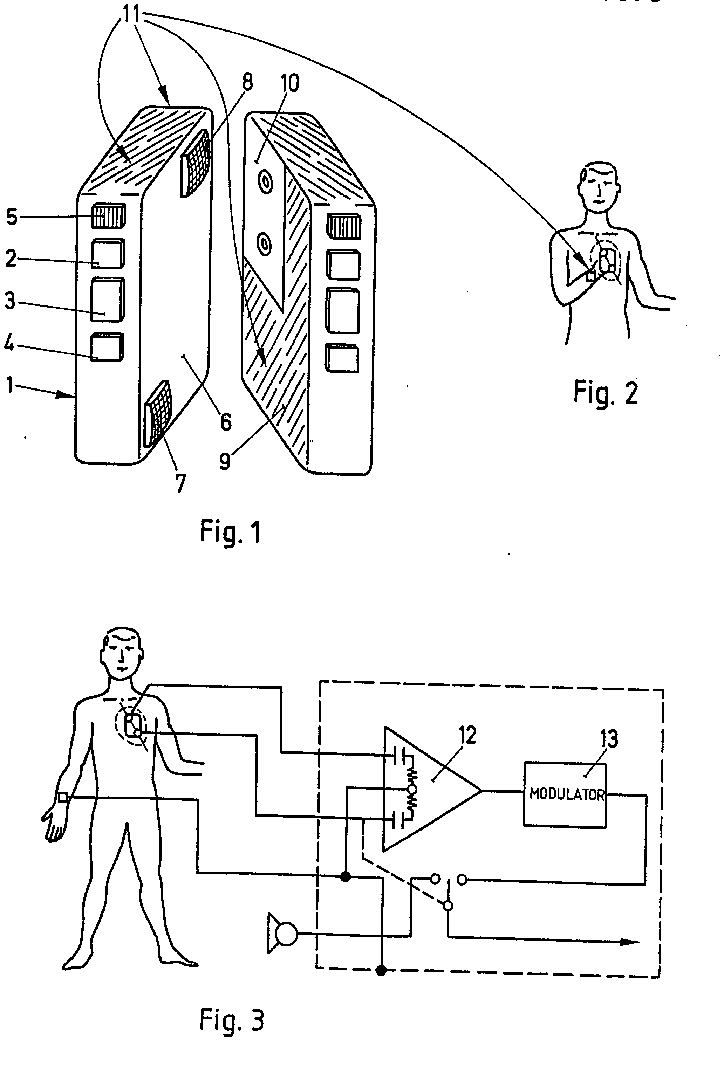

Das in Fig. 1 perspektivisch aus zwei Richtungen gezeigte Gerät 1 ist im wesentlichen ein modifiziertes Kassetten-Magnetbandaufzeichnungsgerät, wie es als Diktiergeräte im Handel erhältlich ist (z.B. Sony M9). Auf seiner Längsseite befinden sich die Betätigungstasten für Aufzeichnung 2, Abspielen 3 und Stop 4. Selbstverständlich können die Betätigungstasten auch anders angeordnet und kombiniert werden. So ist es beispielsweise möglich auf die Stoptaste 4 zu verzichten. Oberhalb der Betätigungstasten ist ein Mikrofon 5 eingebaut. Ueber das Mikrofon 5 kann der Benutzer das Magnetband besprechen und bei dem hier vorgesehenen Einsatzzweck beispielsweise eine Beschreibung seiner Symptome festhalten, nachdem er die Aufzeichnung des EKG beendet hat.

Auf der einen Flachseite 6 des Geräts sind zwei Körperelektroden 7, 8 in zwei einander diagonal gegenüberliegenden Ecken der Seitenfläche angeordnet. Bei diesen beiden Elektroden handelt es sich um die eigentliche Messelektrode 7 und eine Referenzelektrode 8. Die Anordnung diagonal gegenüber wurde gewählt, damit die Elektroden möglichst weit voneinander entfernt sind, um eine für die EKG-Aufzeichnung möglichst gute Differentialbasis zu erzielen.

Auf der gegenüberliegenden Flachseite 9 des Geräts befindet sich der Einschubteil 10 für die Magnetbandkassette, der mit dem von handelsüblichen Diktiergeräten vollständig identisch ist.

Als weitere Modifikation gegenüber einem handelsüblichen Diktiergerät besitzt das in Fig. 1 gezeigte Gerät ausser den beiden Elektroden 7, 8 eine dritte Elektrode 11 in Form einer leitenden Beschichtung des Gehäuses. Diese dritte Elektrode dient als Erdungselektrode. Der Kontakt zwischen dem Patienten und dieser Erdungselektrode 11 wird dadurch hergestellt, dass der Patient das Gerät in seiner rechten Hand hält, während er es mit der Flachseite 6 und den Elektroden 7, 8 gegen seine Brust drückt wie das in Fig. 2 gezeigt ist. Auf diese Weise besteht ein verhältnismässig grosser Abstand zwischen Messelektrode 7 und Referenzelektrode 8 einerseits und der Erdungselektrode 11 andererseits. Dadurch wird erreicht, dass trotz der Anordnung von allen drei Elektroden am Gehäuse der Abstand zwischen dem Erdungspunkt und den beiden Messpunkten praktisch gleich gross ist wie bei der herkömmlichen EKG-Aufzeichung (diagnostische Bandbreite 0,05-100 Hz).

Eine weitere Besonderheit besteht, wie in Fig. 3 gezeigt, darin, dass im Gerät ein herkömmlicher EKG-Verstärker 12 mit Modulator 13 enthalten ist, mit dem die beiden Elektroden 7, 8 und die Erdungselektrode 11 verbunden sind und dessen Ausgangssignale dem Aufzeichnungskopf des Magnetbandgerätes zugeführt werden können.

Zur Inbetriebnahme legt der Patient bzw. die Testperson das Gerät flach in die Hand, so dass die Betätigungstasten mit den Fingern bedient werden können und gleichzeitig die Erdungselektrode 11 von der Handfläche bzw. vom Handballen kontaktiert wird. Sodann wird das Gerät wie in Fig. 2 gezeigt gegen die linke Brustseite gedrückt und die Bandaufzeichnung in Gang gesetzt. Nach einer kurzen Aufzeichnungsperiode, wenn beispielsweise die Symptome wieder aufgehört haben, kann der Patient bzw. die Testperson über das Mikrofon 5 noch eine Beschreibung der Symptome auf das Band sprechen. Anschliessend kann das Gerät bis zur Aufzeichnung eines nächsten Vorkommnisses versorgt werden.

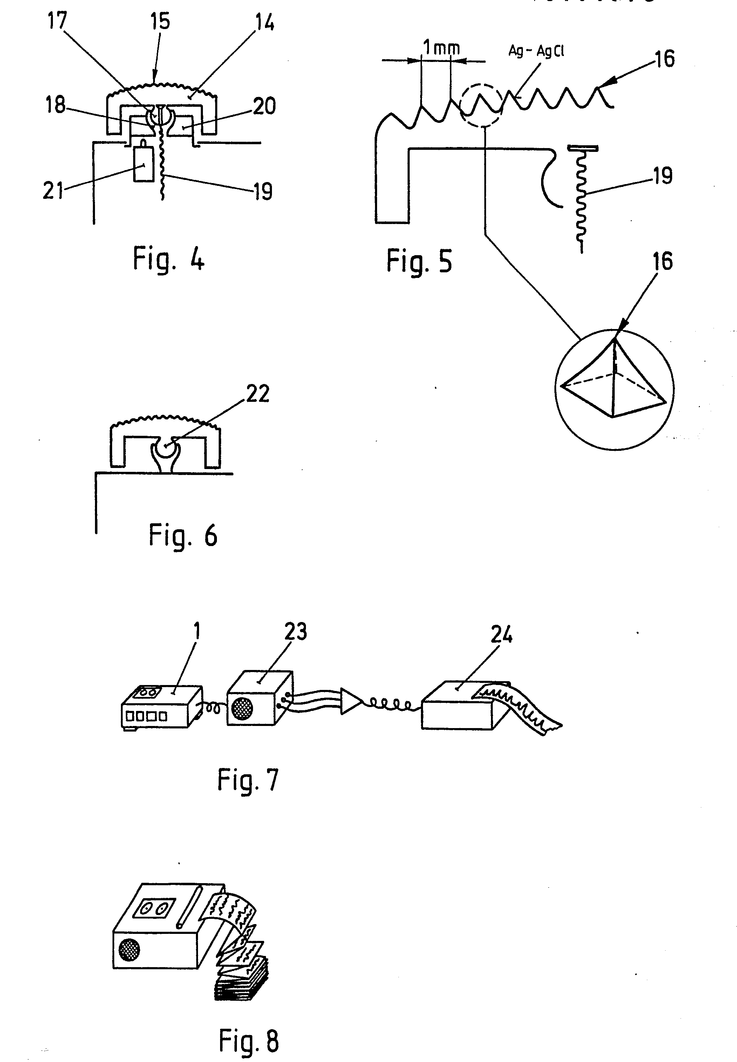

In den Figuren 4 und 5 ist eine bevorzugte Ausführungsform der Messelektrode 7 gezeigt. Ein deckelförmiger Teil 14 besitzt eine gewölbte obere Fläche 15 die zum Auflegen auf der Haut des Patienten dient. Zur Verbesserung des Kontakts ist diese Fläche mit einer Vielzahl von prismatischen Spitzen 16 versehen, die in Fig. 5 vergrössert gezeigt sind. Der Raster dieser Spitzen beträgt etwa einen Millimeter. Die Oberfläche der Spitzen ist mit Silber/ Silberchlorid beschichtet, um Polarisationspotentiale zu verhindern.

In der Mitte der inneren Unterseite des Teils 14 befindet sich ein Vorsprung 17, der einen kugelförmigen Kopf bildet. Dieser kugelförmige Kopf bildet zusammen mit einem hohlkugeligen Gegenstück 18 am Gehäuse des Aufzeichnungsgeräts ein Kugelgelenk, das eine gewisse Beweglichkeit der Elektrode zur Anpassung an die Krümmung der Körperoberfläche des Patienten bewirkt. Vom kugelförmigen Kopf führt eine vorzugsweise spiralig angeordnete Verbindungleitung 19 zum Eingang des EKG-Verstärkers 12 (Fig. 3).

Die Pfanne 18 des Kugelgelenks kann entweder fest am Gehäuse angebracht sein oder wie im vorliegenden bevorzugten Ausführungsbeispiel an einem senkrecht zur Gehäuseoberfläche verschiebbaren Wandteil 20. Auf der Innenseite des verschiebbaren Wandteils 20 ist ein Druckschalter 21 angeordnet, der durch Eindrücken des Wandteils 20 in das Gehäuse betätigt wird. Der Schalter 21 (vgl. auch Fig. 3) ist so zwischen den Ausgang des EKG-Verstärkers 12 und den

Aufzeichnungskopf des Magnetbandgeräts geschaltet, dass die EKG-Signale aufgezeichnet werden, wenn durch den Anpressdruck des Geräts an den Körper des Patienten die Elektrode 7 und der verschiebbare Wandteil 20 auf den Schalter 21 drücken. Auf diese Weise wird sichergestellt, dass für die Aufzeichnung ein gewisser Mindestdruck auf die Elektrode 7 wirkt und damit ein genügend guter Kontakt für die EKG-Aufzeichnung hergestellt ist. Wenn auf die Elektrode kein Druck ausgeübt und damit der Schalter 21 nicht betätigt wird, kann das Magnetband über das Mikrofon besprochen werden.

Da die Referenzelektrode 8 nicht ebenfalls mit einem Druckschalter versehen sein muss, ist sie, wie in Fig. 6 gezeigt, einfacher aufgebaut als die Elektrode 7. Vorzugsweise ist sie in ähnlicher Art und Weise durch ein Kugelgelenk 22 mit dem Gehäuse verbunden, wobei jedoch das Gegenstück des Kugelgelenks in diesem Fall fest am Gehäuse angebracht ist. Abgesehen von der fehlenden zusätzlichen Ausbildung zur Betätigung eines Druckschalters ist somit die Elektrode 8 gleich aufgebaut wie die Elektrode 7.

Um die Auswertung des auf Magnetband aufgezeichneten EKG-Signals zu ermöglichen, wird das Signal in ein optisches Signal umgewandelt. Gleichzeitig werden die auf Band gesprochenen Informationen abgehört. Zu diesem Zweck eignet sich eine Anordnung, wie sie in Fig. 7 gezeigt ist. Das Gerät 1 wird direkt an ein Wiedergabegerät 23 angeschlossen, das einen Demodulator und einen Lautsprecher enthält und mit dem die auf das Band gesprochenen Notizen abgehört werden können. Der Ausgang des Demodulators ist mit einem handelsüblichen Elektrokardiographen 24 verbunden, der das EKG-Signal in der üblichen Art und Weise aufzeichnet.

Anstatt das Gerät 1 direkt an das Wiedergabegerät 23 anzuschliessen, kann selbstverständlich auch ein spezielles Abspielgerät benutzt werden. Dies ist beispielsweise dann sinnvoll, wenn die bespielte Kassette dem behandelnden Arzt zur Evaluation zugeschickt wird.

Eine andere Möglichkeit für die Aufzeichnung des EKG's ist in Fig. 8 gezeigt. Bei dem hier schematisch dargestellten Gerät handelt es sich um einen Hochgeschwindigkeitsschreiber mit sog. Thermopapier (z.B. RICOH TH 3104), der die Aufzeichnung in kompakter Form vornimmt. Dieses Gerät hat ebenfalls einen Lautsprecher zum Abhören der auf das Band gesprochenen Bemerkungen.

Das vorstehend beschriebene Gerät hat den grossen Vorteil, dass es vom Patienten über längere Zeit, beispielsweise mehrere Monate, unbenutzt mitgenommen werden kann, bis ein aufzeichnungswürdiger Vorfall eintritt. Dies ist mit den bisher bekannten sogenannten Holter-Geräten nicht möglich gewesen, weil der Kontakt der herkömmlichen EKG-Elektroden nach spätestens 72 Stunden durch Fachpersonal erneuert werden muss. Ausserdem wäre es für den Patienten unzumutbar, während langer Zeit EKG-Elektroden zu tragen, wenn es völlig unbestimmt ist, wann eine signifikante Aufzeichnung möglich ist.

Eine ähnlich bequeme Handhabung war bisher nur mit den für die telephonische Uebertragung geeigneten Geräten möglich, die jedoch nur unter ganz bestimmten Voraussetzungen eingesetzt werden können und ausserdem eine Aufzeichnung von vergleichsweise minderer Qualität ergeben. Demgegenüber stellt das hier vorgeschlagene Gerät ein vollwertiges EKG-Diagnoseinstrument dar, das dem Arzt alle für die Beurteilung des EKG's notwendigen Informationen liefert. Insofern ist also das hier vorgeschlagene Gerät den Geräten zur telephonischen Uebertragung der Signale überlegen.

Gemäss einer weiteren (nicht gezeigten) Ausführungsform der Erfindung ist das Bandaufzeichnungsgerät vom EKG-
Verstärkerteil getrennt ausgebildet,      wobei das Bandaufzeichnungsgerät wie ein Modul in ein den EKG-Verstärker
enthaltendes Gehäuse einschiebbar bzw. einsteckbar ist.
Dieses Gehäuse besitzt die Elektroden und erforderlichen
Schalter. Als Bandaufzeichnungsgerät für diese Ausführungsform eignet sich beispielsweise das Gerät Sony M-100.

Dieses Gerät besitzt auch einen Lautsprecher, der sich
in besonders eiligen Fällen auch zur telephonischen Uebermittlung der Aufzeichnung eignet.

## Patentansprüche

1. Tragbares Elektrokardiologiegerät mit Körperelektroden zur Erfassung von Herzaktionssignalen, Verstärker und Signalumformer zur Umformung der erfassten Signale in zur Aufzeichnung auf Magnetband geeignete Signale und einem Magnetbandaufzeichnungsteil, dadurch gekennzeichnet, dass die Körperelektroden (7, 8, 11) direkt am Gehäuse des Aufzeichnungsteils angebracht sind.

2. Elektrokardiologiegerät nach Anspruch 1, dadurch gekennzeichnet, dass die Körperelektroden Mess- (7), Referenz- (8) und Erdungselektroden (11) umfassen und dass Mess- und Referenz-Elektroden an einer ersten Wand (6) des Geräts im Abstand voneinander angebracht sind.

3. Elektrokardiologiegerät nach Anspruch 2, dadurch gekennzeichnet, dass die Erdungselektrode an einer von der ersten verschiedenen Wand (9) des Geräts angeordnet ist.

4. Elektrokardiologiegerät nach Anspruch 1, dadurch gekennzeichnet, dass eine der Elektroden (7) mit einem Druckschalter (21) integriert ist.

5. Elektrokardiologiegerät nach Anspruch 1, dadurch gekennzeichnet, dass zusätzlich ein Mikrofon (5) zur Aufzeichnung akustischer Signale vorhanden ist.

***

1/2

0101870

Fig. 1

Fig. 2

Fig. 3

Fig. 4

Fig. 5

Ag – AgCl

1mm

Fig. 6

Fig. 7

Fig. 8